# EUROPÄISCHE PATENTSCHRIFT

(11) **EP 0 312 645 B1**
(45) Veröffentlichungstag und Bekanntmachung des Hinweises auf die Patenterteilung: **15.09.1993**
(21) Anmeldenummer: 87115617.0
(22) Anmeldetag: 23.10.1987
(51) Int. Cl.: C07K 7/06, A61K 37/02, C12N 15/00, C12P 21/00, G01N 33/53, A61K 39/385, A61K 39/395

(54) **Antigene Peptide des Komplementfaktors C3a, Verwendung der Peptide, Zell-Linien, Antikörper gegen die Peptide und Verwendung der Antikörper**
Antigenic peptides from complement factor C3a, use of peptides, cell lines, antibodies against the peptides and utilisation of the antibodies
Peptides antigéniques du facteur C3a du complément, utilisation des peptides, lignées cellulaires, anticorps contre les peptides et utilisation des anticorps

(43) Veröffentlichungstag der Anmeldung: 26.04.1989
(73) Patentinhaber: PROGEN Biotechnik GmbH, 69120 Heidelberg (DE)
(72) Erfinder: Naser, Werner, Dr. Dipl.-Biol., D-6915 Dossenheim (DE); Burger, Reinhard, Dr. Prof., D-6900 Heidelberg (DE)
(74) Vertreter: Grünecker, Kinkeldey, Stockmair & Schwanhäusser Anwaltssozietät

(56) Entgegenhaltungen:
- EP-A- 0 097 440
- DE-A- 2 803 238
- PROCEEDINGS OF THE NATIONAL ACADEMY OF SCIENCES OF THE USA, Band 82, Nr. 3, Februar 1985, Seiten 708-712; M.H.L. DE BRUIJN et al.: "Human complement component C3: cDNA coding sequence and derived primary structure"
- THE JOURNAL OF IMMUNOLOGY, Band 131, Nr. 5, November 1983, Seiten 2258-2261, The American Association of Immunologists, San Diego, US; E.L. MORGAN et al.: "Suppression of humoral immune responses by synthetic C3a peptides"
- CHEMICAL ABSTRACTS, Band 107, Nr. 11, 14. September 1987, Seite 542, Zusammenfassung Nr. 94949u, Columbus, Ohio, US; R. BURGER et al.: "Functional analysis and quantification of the complement C3 derived anaphylatoxin C3a with a monoclonal antibody", & CLIN. EXP. IMMUNOL. 1987, 68(3), 703-11
- BIOCHEMISTRY, Band 23, Nr. 4, 14. Februar 1984, Seiten 585-588, American Chemical Society; G. UNSON et al.: "Active site of C3a anaphylatoxin: contributions of the lipophilic and orienting residues"
- CHEMICAL ABSTRACTS, Band 100, Nr. 7, 13. Februar 1984, Seite 428, Zusammenfassung Nr. 49713g, Columbus, Ohio, US; J.L. WAGNER et al.: "Radioimmunoassay for anaphylatoxins: a sensitive method for determining complement activation products in biological fluids", & ANAL. BIOCHEM. 1984, 136(1), 75-88
- CHEMICAL ABSTRACTS, Band 87, Nr. 7, 15. August 1977, Seite 326, Zusammenfassung Nr. 51465j, Columbus, Ohio, US; T.E. HUGLI et al.: "Synthetic peptides with the biological activities and specificity of human C3a anaphylatoxin", & PROC. NATL. ACAD. SCI. U.S.A. 1977, 74(5), 1826-30

## Beschreibung

Die Erfindung betrifft ein Peptid, die Verwendung des Peptides zum Immunisieren eines Tieres, Antikörper gegen das Peptid, Zell-Linien und die Verwendung von Antikörpern.

Zahlreiche Erkrankungen gehen mit einer Aktivierung des Komplementsystems einher. Der Nachweis eines Produktes der Komplementaktivierung liefert dann einen Hinweis darauf, daß eine Komplementaktivierung abläuft oder aber abgelaufen ist. Daraus ergeben sich Rückschlüsse zum Pathomechanismus, zur Differentialdiagnose, Beurteilung des Schweregrades, Prognose, Therapieerfolg etc. Der Nachweis einer Komplementaktivierung auf der Grundlage der Bestimmung eines wichtigen Komplementfaktors, z.B. des Faktors C3a, erfolgt beispielsweise bei Krankheitsbildern, bei denen Immunkomplexe beteiligt sind, da diese in der Lage sind, Komplement zu aktivieren. Derartige Ereignisse sind z.B. bei der rheumatoiden Arthritis, Serumkrankheit und systemischem Lupus erythematodes nachgewiesen worden.

Ein Schlüsselmolekül der proteolytischen Komplementaktivierungs-Kaskade ist der Komplementfaktor C3.

C3 besteht aus zwei Untereinheiten, der α - und der β -Kette. Der vollständige Faktor hat ein Molekulargewicht von 180000 Dalton. Bei der Aktivierung von C3 kommt es zu einer Spaltung der α -Kette zwischen den Aminosäuren 77 und 78. Durch die Spaltung entstehen ein kleines Fragment, C3a, das eines der sog. Anaphylatoxine ist, und ein größeres Fragment, C3b.

Das durch die Spaltung erzeugte kleinere Bruchstück C3a ist biologisch hoch aktiv und löst auf der zellulären Ebene nach Bindung an entsprechende C3a-Rezeptoren auf verschiedenen Zellen (z.B. Granulozyten, Makrophagen, Thrombozyten, Mastzellen, Basophile) diverse Reaktionen aus. C3a besteht aus 77 Aminosäuren und hat ein Molekulargewicht von 9000 Dalton. Der aktive Bereich des Moleküls befindet sich in dem äußersten C-terminalen Hexapeptid, d.h. den Aminosäuren 72-77. Dieser Abschnitt entspricht dem C3a-Bereich unmittelbar vor der Spaltstelle innerhalb des C3-Moleküls. Für die biologische Aktivität von C3a ist das terminale Arginin in Position 77 von Bedeutung. Unter physiologischen Bedingungen wird dieses Arginin durch die ubiquitäre Serum-Carboxypeptidase N (Anaphylatoxin-Inaktivator) abgespalten und C3a dadurch schnell inaktiviert. Auf diese Weise entsteht das sog. C3a-desArg, dessen terminale Aminosäure das Alanin aus Position 76 ist. C3a/C3a-desArg ist biochemisch gut charakterisiert und läßt sich durch standardisierte Reinigungsverfahren aus aktiviertem Serum isolieren.

Die quantitative Erfassung von C3a ist z.B. bei Patienten mit "Adult Respiratory Distress Syndrome" (ARDS) von diagnostischer Bedeutung. C3a ist einer der wenigen Laborparameter mit prognostischem Wert. Der C3a-Anstieg bei ARDS-Patienten erfolgt unabhängig von der Art der Komplementaktivierung; diese kann sowohl über den klassischen Weg (z.B. bei immunkomplexabhängigen Erkrankungen) erfolgen, als auch über den alternativen Weg (z.B. künstliche Membran bei Bypass-Operationen, Sepsis) als auch nach direkter Spaltung von C3 durch Proteasen (z.B. Polytrauma, Pankreatitis).

Bereits bekannte Testsysteme, die auf der biologischen Funktion von C3a beruhen, lassen sich zur Quantifizierung von C3a in Patientenserum oder -plasma nicht einsetzen, da die Funktion von der Präsenz des C-terminalen Arginins in Position 77 von C3a abhängig ist. Dieses wird jedoch unter Serum-Bedingungen durch die ubiquitäre Carboxypeptidase N abgespalten, wodurch das inaktive C3a-desArg entsteht. Aus diesem Grunde sind serologische Nachweis-Systeme etabliert worden, die sowohl C3a als auch C3a-desArg erfassen. In der klinischen Diagnostik wird dabei ein Radioimmunoassay eingesetzt, der auf der Kompetition von endogenem C3a in der zu messenden Probe mit einem radioaktiv markierten C3a bzw. C3a-desArg um die Antikörper-Bindungsstelle eines anti-C3a/C3a-desArg-Serums beruht. Das dabei üblicherweise verwendete Radioisotop ¹²⁵J hat allerdings eine Halbwertszeit von nur ca. 2 Monaten und ist deshalb nur begrenzte Zeit verwendbar. Darüberhinaus sind beim Arbeiten mit ¹²⁵J die Vorschriften der Strahlenschutzverordnung zu beachten, die eine adäquate Laborausrüstung erfordern. Die Bestimmung von C3a auf der Basis von Testsystemen, die ohne Radioaktivität auskommen, wäre daher zu bevorzugen.

Vor kurzem wurde erstmals ein C3a-Nachweis auf der Basis eines ELISA-Systems unter Verwendung eines monoklonalen Antikörpers beschrieben (Burger et al., Clin.exp. Immunol. (1987), 68, 703-711). Sowohl dieser ELISA als auch der Radioimmunoassay haben jedoch den grundsätzlichen Nachteil, daß hierbei Antiseren oder monoklonale Antikörper gegen den natürlichen Faktor C3a verwendet werden, die gegen solche antigenen Determinanten gerichtet sind, die auch auf dem nicht-aktivierten Komplementfaktor C3 exponiert sind und daher zur Reaktion der Antikörper auch mit dem ungespaltenen Protein führen.

Eine C3a-Bestimmung unter Verwendung dieser Antikörper setzt daher eine Vorbehandlung der biologischen Flüssigkeit, in der der Komplementfaktor C3a bestimmt werden soll, voraus. In der Praxis bedeutet dies die Durchführung von Präzipitationsreaktionen mit geeigneten fällungsmitteln, um das ungespaltene C3 möglichst quantitativ aus der zu messenden Probe zu entfernen. Der damit verbundene technische Aufwand verteuert und verzögert die Bestimmung des Faktors C3a. überdies verfälschen von der Fällung nicht erfasste Restmoleküle C3 das Ergebnis.

Eine Aufgabe der vorliegenden Erfindung war es, Mittel bereitzustellen, die die Erzeugung von C3a spezifischen Antikörpern ermöglichen.

Diese Aufgabe wird erfindungsgemäß gelöst durch ein Peptid mit der Aminosäureseuqenz H₂N-Arg-Ala-Ser-His-Leu-Gly-Leu-Ala-COOH bzw. einem Konjugat aus einem Trägermolekül und dem genannten Oktapeptid.

Eine weitere Aufgabe der Erfindung bestand in dem Bereitstellen von C3a-spezifischen Antikörpern.

Diese Aufgabe wird erfindungsgemäß gelöst durch Antikörper gegen das oben genannte Peptid oder gegen das entsprechende Konjugat, wobei dei Antikörper erhältlich sind durch Immunisieren eines Tieres mit dem Oktapeptid bzw. dem entsprechenden Konjugat.

Es werden also Antigene zur Verfügung gestellt, die zur Bildung hochspezifischer Antikörper gegen C3a führen und somit die exakte Bestimmung von C3a-Molekülen gestatten.

Zwar ist aus den Arbeiten von Hui et al. (Science 222, 1129, 1983) bereits bekannt, durch Immunisieren mit einem synthetischen Peptid, das dem N-terminalen Teil des Fibrinmoleküls entspricht, fibrinspezifische Antikörper zu erzeugen, die nicht mehr mit dem Vorläufermolekül Fibrinogen kreuzreagieren. Im Falle des Fibrins stand jedoch ein hochantigenes N-terminales Peptid zur Verfügung, das allein 4 geladene Aminosäuren in dem zur Immunisierung verwendeten Bereich enthält, so daß die Antigenizität sehr wahrscheinlich war. Die erfindungsgemäß vorliegende Aminosäuresequenz erfüllt diese Voraussetzung nicht. Aus diesem Grunde war nicht anzunehmen, daß wenn, wie im vorliegenden Fall, ungeladene oder gar hydrophobe Aminosäuren durch die Spaltung freigesetzt werden, ein ähnlicher Effekt erzielt werden könnte.

Die Verwendung der erfindungsgemäßen Peptide hat den Vorteil, daß durch die Wahl einer geeigneten Sequenz durch Immunisieren mit diesem Peptid Antikörper erzeugt werden können, die ganz spezifisch mit dem in diesem Peptid exponierten Hapten reagieren. Das bedeutet, daß beispielsweise im vorliegenden Fall durch die Wahl eines ausschließlich im Komplementfaktor C3a exponierten Epitops für die Sequenz des Peptides eine Reaktion mit dem Faktor C3 und freien Faktor C3b ausgeschlossen werden kann.

Es ist daher sinnvoll, die für die Immunisierung vorgesehenen Peptide so auszuwählen, daß sie Sequenzen enthalten, die erst beim Übergang vom Gesamtmolekül zum gespaltenen Molekül, z.B. durch Konformationsänderungen, antigene Eigenschaften erlangen. Überraschenderweise hat es sich gezeigt, daß auch die Sequenzen in der Nähe der Spaltstelle erst durch die Spaltung immunogen werden. Im Gegensatz dazu hätte der Fachmann erwartet, daß die der Protease zugängliche Spaltstelle auch vor der Spaltung einer Antikörpererkennung zugänglich ist.

Weiterhin ist es höchst überraschend, daß durch Immunisieren mit obigem Peptid eine spezifische Antigen-Antikörper-Wechselwirkung nur zwischen den Antikörpern und Faktor C3a und/oder C3a-desArg stattfindet. Es konnte keinerlei Kreuzreaktion mit dem nativen Molekül C3 beobachtet werden. Dies ist deshalb umso überraschender, als frühere Versuche, monoklonale Antikörper mit dem natürlichen Molekül C3a als Immunogen zu erzielen, zu keinem C3a-spezifischen Antikörper führten. Alle auf diese Art erzielten Antikörper wiesen Kreuzreaktionen mit dem nativen C3-Molekül auf.

Durch das erfindungsgemäße Peptid wird also ein besseres Antigen zur Verfügung gestellt, das eine hohe Spezifität der entsprechenden monoklonalen Antikörper erzeugt und als Neoantigen zu bezeichnen ist.

Es ist bekannt, daß der Komplementfaktor C3a in vivo relativ schnell durch Carboxypeptidase N inaktiviert wird, indem das C-terminale Arginin abgespalten wird. Die durch diese Spaltung gebildeten Fragmente C3a-desArg bzw. Teile davon eignen sich überraschenderweise ebenso zum Immunisieren; auch ihre Sequenz ist spezifisch für erst durch die Spaltung freigelegte Bereiche des Komplementfaktors C3a.

Sinnvollerweise wird für die Erzeugung eines Antikörpers gegen den Komplementfaktor C3a ein Peptid aus dem C-terminalen Bereich des Komplementfaktors C3a oder C3a-desArg verwendet, das neben einigen neutralen Aminosäuren die Aminosäuren Arginin und Histidin enthält (Arg 69 und His 72). Es wurde das Octapeptid H₂N-Arg-Ala-Ser-His-Leu-Gly-Leu-Ala-COOH verwendet.

Das erfindungsgemäße Peptid kann auf verschiedene Weise hergestellt werden. Eine erste Möglichkeit wäre die Verwendung von natürlichen Peptiden, die durch Proteolyse des gereinigten Faktors C3a bzw. C3a-desArg oder auch des gereinigten Komplementfaktors C3 gewonnen werden. Dem Fachmann sind inzwischen eine Vielzahl von Proteasen sowie von chemischen Reaktionen, die zu definierten Spaltungen führen, bekannt, mit deren Hilfe Subfragmente aus dem Bereich des Komplementfaktors C3a hergestellt werden können, die anschließend durch herkömmliche Methoden, beispielsweise unter Verwendung von HPL-Chromatografie, gereinigt werden können.

Eine bevorzugte Möglichkeit ist die Bereitstellung der Pepitde durch chemische Synthese, z.B. durch die Merrifield-Synthese.

Die Verwendung von synthetischen Peptiden als Antigene hat gegenüber der Verwendung natürlich isolierter Komplementfaktoren oder Komplementfaktorfragmente zwei ganz wesentliche Vorteile. So lassen sich nach den bekannten Syntheseverfahren, z.B. der Merrifield-Synthese, synthetische Peptide oder Polypeptide in ausreichend großem Maßstab und in hoher Reinheit herstellen, so daß die aufwendigen Verfahren zum Isolieren und Reinigen des natürlichen Proteines umgangen werden. Darüberhinaus ist die Reinigung synthetischer Peptide ein gut etabliertes Verfahren, während auch ein technisch aufwendiges Anreicherungs- und Reinigungsverfahren für natürliche Proteine stets zu Präparationen führt, die einen zwar unbestimmbar kleinen, aber doch als Antigen wirksamen Anteil unerwünschter Peptide enthalten können.

Die Herstellung gewünschter Peptide mit gentechnischen Methoden ist ebenso möglich. Dabei wäre eine Expression des erfindungsgemäßen Peptides in Prokaryonten oder niederen Eukaryonten bevorzugt, um die Kontamination des erzeugten Peptides mit Proteinen höherer Eukaryonten zu vermeiden.

Die erfindungsgemäßen Peptide werden vor der Verwendung zum Immunisieren sinnvollerweise an Trägermoleküle gekoppelt. Dabei werden die für den C-Terminus des Komplementfaktors C3a spezifischen Peptide bevorzugt N-terminal gekoppelt. Durch diese Art der Kopplung bleibt das geschaffene Neoantigen für eine Antigen-Antikörper-Wechselwirkung frei verfügbar. Im Fall desjenigen Peptides, das N-terminal ein Arginin enthält, liegen im gesamten Molekül nur eine freie Amino- und eine freie Guanidinogruppe, beide vom N-terminalen Arginin, vor. Die orientierte Bindung dieses Peptides kann beispielsweise unter Verwendung von Glutardialdehyd durchgeführt werden, was zur kovalenten gerichteten Bindung der Amino- oder Guanidinogruppe eben dieses Arginins an das gewünschte Trägermolekül führt. Das jeweilige Kopplungsverfahren kann dabei oft im großen Maß variiert werden. So können z.B. bei der Verwendung von Glutardialdehyd sogenannte Einschritt- (Träger und Peptid werden gleichzeitig dem Glutardialdehyd ausgesetzt) oder Zweischrittmethoden (Träger oder Peptid werden zunächst aktiviert und dann gemischt) angewendet werden. Das gleiche gilt bei der Verwendung von anderen Kopplungsreagentien. Darüberhinaus kann die Konzentration an Kopplungsreagenz in einem weiten Bereich variiert werden. Die Konzentration von Glutardialdehyd liegt z.B. zwischen ca. 0,01 % - ca. 2 %. Nach erfolgter Kopplungsreaktion werden in an sich bekannter Weise nicht umgesetzte aktive Aldehydgruppen durch Zugabe eines Überschusses an reaktiven Aminogruppen inaktiviert. Die Trägerprotein-Peptidkonjugate werden anschließend durch übliche Maßnahmen wie Dialyse oder chromatografische Methoden von noch vorhandenen niedermolekularen Verunreinigungen abgetrennt und dabei in einen physiologischen Puffer überführt.

Das erfindungsgemäße Peptid kann an synthetische oder natürliche Polymere gekoppelt werden. Bevorzugte synthetische Polymere sind z.B. bromcyanaktivierte Sepharosematerialien, die darüberhinaus die Verwendung der trägergekoppelten Peptide als Matrix für Affinitätschromatografie gestatten. Erfindungsgemäß verwendbare natürliche Polymere sind z.B. Polysaccharide oder Proteine.

Bevorzugte natürliche Trägermoleküle sind z.B. Albumine oder Hämocyanine. Zweckmäßigerweise werden dabei solche Proteine als Träger ausgewählt, die keinerlei immunologische Verwandtschaft zum bearbeiteten Antigen aufweisen.

Das erfindungsgemäße Peptid wird für die Immunisierung von Tieren verwendet, um so die Bildung von spezifisch gegen des Peptid gerichteten Antikörpern zu induzieren. Für eine exakte Bestimmung ist es bevorzugt, antikörperproduzierende Zellen des immunisierten Tieres durch Fusion mit transformierten Zellen zu immortalisieren. Bevorzugt werden daher für die Immunisierung Mäuse verwendet, denen nach erfolgter Immunisierung beispielsweise Thymus- oder Milzzellen entnommen werden.

Durch das Immunisieren mit dem erfindungsgemäßen Peptid werden Antikörper veschiedener Spezifität gebildet. Dabei hat es sich überraschenderweise gezeigt, daß bei der Immunisierung von Tieren mit dem C-terminalen Peptid des Komplementfaktors C3a eine ganz spezifische Reaktion mit C3a-typischen Sequenzen stattfindet. Überraschenderweise reagiert ein Großteil solcher Antikörper nicht mit dem Gesamtprotein C3.

Die durch Immunisieren von Mäusen oder beispielsweise Kaninchen erhaltenen Antikörper sind eine Mischung verschiedener polyklonaler Antikörper. Aus den diese Antikörper enthaltenden Seren können die spezifisch mit dem zur Immunisierung eingesetzten Peptid reagierenden Antikörper z.B. durch Affinitätschromatografie an Träger gekoppelte Peptide nach Anspruch 7 gereinigt werden.

Ein Nachteil polyklonaler Antikörper ist jedoch, daß die sie enthaltenden Seren nicht stets mit gleicher Qualität erzeugt werden können. Versuche mit polyklonalen Antikörpern sind nur dann ohne weiteres miteinander vergleichbar, wenn die zu den Versuchen verwendeten Seren aus der gleichen Charge stammen.

Die Verwendung monoklonaler Antikörper ist daher bevorzugt. Monoklonale Antikörper können nach dem Fachmann bekannten Verfahren hergestellt werden, indem z.B. Milz- oder Thymuszellen eines gegen das jeweilige Peptid immunisierten Tieres mit transformierten Zellen fusioniert werden. Dafür wird bevorzugt eine Myelomzell-Linie verwendet, die nicht mehr in der Lage ist, selber Immunglobuline zu synthetisieren. Aus der Vielzahl der fusionierten, in HAT-Selektionsmedium angewachsenen Zellen müssen dann die Zell-Linien herausgesucht werden, die die wunschgemäßen Antikörper produzieren. Auf diese Weise wurden Antikörper erhalten, die spezifisch mit den Komplementfaktoren C3a und/oder C3a-desArg reagierten.

Die Spezifität der jeweiligen Antikörper wurde getestet, indem der Kulturüberstand einer zweifach klonierten Hybridomazellinie gewonnen und im sogenannten indirekten ELISA mit verschiedenen Antigenen inkubiert wurde. Dabei zeigte es sich, daß die beiden isolierten monoklonalen Antikörper H453 und H454 insofern gleichartig reagierten, als sie deutlich mit Komplementfaktor C3a, mit desaktiviertem Komplementfaktor C3a (C3a-desArg) und mit Octapeptid-gekoppelten Proteinen, z.B. Octapeptid-Rinderserumalbumin, reagierten. Hingegen reagierten beide Antikörper nicht mit nativem ungespaltenem Komplementfaktor C3, beide Antikörper reagierten nicht mit unmodifiziertem Rinderserumalbumin oder Keyhole Limpet Hämocyanin.

Die Detektion der jeweils gebundenen Antikörper erfolgte jeweils durch Inkubation mit einem zweiten, mit einem Markerenzym konjugierten Antikörper. In einer bevorzugten Ausführungsform wird zum Nachweis des gebundenen Mausantikörpers ein peroxidasekonjugierter Kaninchen-Anti-Mausantikörper verwendet. In dem Fachmann an sich bekannter Weise werden anschließend nicht gebundene Antikörper entfernt und die Menge gebundener Antikörper durch Umsetzung einer Substrat lösung quantifiziert. Die Auswertung der Umsetzung erfolgt in diesem Falle photometrisch. Weitere mögliche Ausführungsformen sind der Nachweis gebundener Mausantikörper durch fluoreszenzmarkierte Anti-Maus-Immunglobuline, deren gebundener Anteil anschließend durch Fluoreszenzphotometrie detektiert wird, oder auch die Verwendung biotinylierter oder radioaktiv markierter Antikörper.

Erfindungsgemäß können die gewonnenen Antikörper zur direkten Bestimmung des gewünschten Komplementfaktors, d.h. Komplementfaktor C3a und/oder C3a-desArg, im Plasma verwendet werden. Die so gewonnenen Antikörper haben den wesentlichen Vorteil, daß sie nur mit dem jeweiligen Bruchstück, nicht jedoch mit dem gesamten nativen Protein C3 reagieren. Daher wird bei der Bestimmung des Anteils von C3a und/oder C3a-desArg im Plasma oder im Serum vermieden, den Anteil noch vorhandenen intakten C3-Komplementfaktors vor Durchführung des Testes präzipitieren zu müssen. Infolgedessen werden die Werte auch nicht durch die möglicherweise unvollständige Fällung beeinflußt. Durch Verwendung der erfindungsgemäßen Antikörper bei der Durchführung der Bestimmung von Komplementfaktor C3a und/oder C3a-desArg steht ein einfaches, schnelles und äußerst zuverlässiges Testverfahren zur Verfügung.

Die erfindungsgemäßen Antikörper können weiterhin als Therapeutikum verwendet werden. Dabei macht man sich zunutze, daß Antikörper gegen die Erkennungssequenz des Peptids nach Anspruch 1 die aktive Stelle des C3a-Moleküls blockieren. Daraus folgt, daß der Antikörper-gebundene Faktor C3a seine biologische Funktion nicht mehr erfüllen kann. Die Verwendung der erfindungsgemäßen Antikörper zur Behandlung von krankhaften Erscheinungen, die auf einer Überaktivierung des Komplementsystemes beruhen, hat eine erhebliche therapeutische Relevanz.

Die folgende Figur und die Beispiele erläutern die Erfindung:

### Figurenbeschreibung:

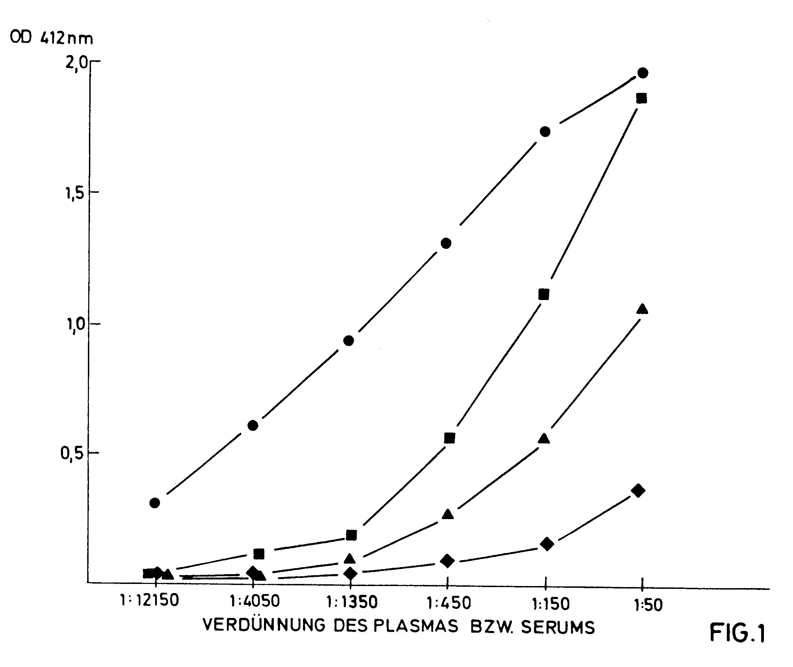

Figur 1 zeigt die Ergebnisse einer erfindungsgemäßen Bestimmung des C3a- bzw. C3a-desArg-Gehaltes verschiedener Serum- und Plasmaproben bei unterschiedlichen Verdünnungen. Dabei bedeuten:
- (●):: zymosanaktiviertes Serum (ca. 4000 ng C3a/C3a-desArg/ml Probe)
- (■):: C3a/C3a-desArg-haltiges Plasma (ca. 1000 ng C3a/C3a-desArg /ml Probe)
- (▲):: C3a/C3a-desArg-haltiges Plasma (ca. 500 ng C3a/C3a-desArg/ml Probe)
- (♢):: EDTA-behandeltes Plasma (ca. 200 ng C3a/C3a-desArg/ml Probe).

Die Durchführung des der Figur zugrundeliegenden Versuches ist in Beispiel 3 d) beschrieben.

### Verwendete Abkürzungen:

- TOS:: Tosyl
- BZL:: Benzyl
- RSA:: Rinderserumalbumin
- KLH:: Keyhole Limpet Hämocyanin
- PBS:: phosphat-gepufferte Saline
- OP:: Octapeptid
- ABTS:: 2,2'-Azino-di-[3-ethylbenzthiazolinsulfonat (6)]

### Beispiel 1: Herstellung des Immunogens

### a) Darstellung des synthetisierten Octapeptids

Ein erfindungsgemäßes Octapeptid mit der Sequenz H₂N-Arg-Ala-Ser-His-Leu-Gly-Leu-Ala-COOH wurde nach der von R. B. Merrifield (J.Amer.Chem. 85, 2148 - 2155, 1963) entwickelten Festphasenmethode unter Verwendung der Protokolle der Fa. Biosearch synthetisiert. Da das Peptid nach der Synthese als Säure vorliegen sollte, wurde ein Merrifieldträger verwendet, an den bereits die erste Aminosäure (hier Alanin) gekoppelt war. Die α -Aminogruppe aller verwendeten Aminosäuren war durch die t-Boc Gruppe geschützt. Zum Schutz der Seitenketten wurden verschiedene Gruppen eingesetzt: im Fall des Arginins und des Histidins die Tosylgruppe, bei Serin die Benzylgruppe. Der dem Fachmann bekannte Synthesezyklus, bestehend aus vier Syntheseschritten, wurde so oft durchlaufen, bis das Peptid die gewünschte Kettenlänge erreicht hatte. Beim erfindungsgemäßen Octapeptid wurde als Träger das Boc-Ala-O-Harz eingesetzt. An diesem wurden, wie im Zyklus beschrieben, nacheinander folgende Aminosäuren synthetisiert: Boc-Leu-OH, Boc-Gly-OH, Boc-Leu-OH, Boc-His(Tos)-OH, Boc-Ser(Bzl)-OH, Boc-Ala-OH, Boc-Arg (Tos)-OH. Die Abspaltung des Peptids vom Träger und die Abspaltung der Schutzgruppen von den Seitenketten erfolgten, wie bei der Merrifieldsynthese üblich, mit flüssiger HF bei 0°C, wobei Amisol als Fänger für das t-Boc-Kation eingesetzt wurde.

Das synthetisierte Peptid wurde anschließend über präparative Reversephase-HPLC bei einem pH-Wert von 2 abgetrennt und mit einem analytischen Lauf auf seine Reinheit überprüft.

Zusätzlich wurde von dem HPLC-gereinigten und lyophilisierten Peptid eine Aminosäureanalyse durchgeführt. Die Werte der Aminosäurenanalyse sind in nachfolgender Tabelle zusammengefaßt:

| **Anzahl** | | |
|---|---|---|
| **Aminosäure** | **gefunden** | **theoretisch** |
| Alanin | 1.8 | 2.0 |
| Arginin | 1.0 | 1.0 |
| Glycin | 1.1 | 1.0 |
| Histidin | 1.0 | 1.0 |
| Leucin | 1.6 | 2.0 |
| Serin | 0.9 | 1.0 |

Die Ergebnisse der Aminosäurenanalyse belegen, daß das synthetisierte Peptid die erwartete Aminosäurenkomposition aufweist.

### b) Herstellung der Peptid-Träger-Konjugate

Die Kopplung an die Trägerproteine (Rinderserumalbumin = RSA und Keyhole limpet haemocyanine = KLH) erfolgte mittels Glutardialdehyd nach einer Zweistufenmethode. Zunächst wurde das jeweilige Trägerprotein mit Glutardialdehyd aktiviert. Trägerprotein (50 mg in 2,5 ml PBS) wurde mit Glutardialdehyd (Endkonzentration 1%) im überschuß versetzt und der Ansatz 2 h bei Zimmertemperatur inkubiert. Zwischenzeitlich wurde eine Sephadex®-G25M Säule (Pharmacia, Art.Nr. 17-0851-01) mit 0,9%iger Kochsalzlösung äquilibriert. Nach Beendigung der Aktivierung wurde das aktivierte Trägerprotein durch Gelchromatographie von freiem Glutardialdehyd gereinigt. Hierzu wurde 1 ml des obigen Ansatzes eingesetzt. Das Eluat (1,5 ml) mit dem aktivierten Trägerprotein wurde mit 5 mg Peptid, das in 0,5 M Na-Carbonat-Puffer (pH 9,5) gelöst war, versetzt. Die Kopplungsreaktion erfolgte für 2 h bei Zimmertemperatur.

Zum Blockieren von nicht umgesetzten Aldehydfunktionen wurde zum Reaktionsansatz anschließend Lysin in einer Endkonzentration von 0,1 M zugegeben. Der Ansatz wurde 30 min bei 37°C inkubiert.

Die Peptid-Trägerprotein-Konjugate wurden mittels Gelchromatographie (Sephadex®-G25M, äquilibriert mit PBS) von nicht umgesetztem Peptid bzw. Lysin abgetrennt und gleichzeitig in einen physiologisch verträglichen Puffer (PBS) überführt.

### Beispiel 2: Herstellung der monoklonalen Antikörper

### a) Immunisieren

Das jeweilige Peptid-Trägerkonjugat (RSA- bzw. KLH-unidirektionell gekoppeltes Peptid) wurde in PBS auf 3,2 mg/ml verdünnt und mit gleichem Volumen Freund'schen Adjuvans emulgiert. Bei der Erstimmunisierung wurde dabei das komplette Freund'sche Adjuvans und bei der Zweitimmunisierung das inkomplette Freund'sche Adjuvans eingesetzt. Jeweils 250 µl des so präparierten Immunogens (ca. 400 ug) wurden ca. 8 Wochen alten Balb/cJ-Mäusen intraperitoneal (i.p.) und subkutan injiziert. Diese Injektionen wurden zweimal im Abstand von je 4-5 Wochen wiederholt. 3 Tage vor der Entnahme der Milz erhielten die Mäuse 400 µg Peptid-Trägerkonjugat (in ca. 120 µl PBS) durch intravenöse Injektion in die Schwanzvene. Tag 2 und Tag 1 vor der Fusion erhielten sie die gleiche Menge an Antigen durch i.p.-Injektion.

### b) Fusion und Selektion

Etwa 5x10⁷ Zellen von der Milz einer immunisierten Maus wurden mit 5x10⁷ Myelomzellen (x63-Ag8-653, eine Linie, die kein Immunglobulin synthetisiert; zu beziehen bei der ATCC) in Gegenwart von Polyethylenglykol (MG 4000) fusioniert. Fusionierte Zellen wurden auf 10 Platten, die jeweils 96 Vertiefungen mit ca. 200 µl Fassungsvermögen enthalten, ausgesät.

Jede dieser Vertiefungen enthielt 2x10⁶ Thymozyten nichtimmunisierter syngener Mäuse in HAT-Selektionsmedium (Nährmedium mit Hypoxanthin-, Aminopterin- und Thymidinzusatz).

Die wachsenden Hybridome wurden zunächst auf Bildung von Antikörpern getestet, die mit gereinigtem C3a-desArg in einem indirekten ELISA reagierten.

Für diesen Test wurde C3a-desArg direkt an PVC-Mikrotiterplatten angelagert. Die Beschichtung mit 50 µl Antigenlösung (5 µg/ml) erfolgte bei Zimmertemperatur für 2 Stunden. Nicht besetzte Proteinbindungsstellen wurden anschließend durch eine zwei-stündige Inkubation mit Rinderserumalbumin (0,5% in PBS) blockiert. Nach dem Waschen der Platten (PBS mit 0,05% Tween 20; 4x) wurden jeweils 50 µl Kulturüberstand der jeweiligen Hybridoma-Kultur für 60 Minuten in den Näpfen inkubiert. Anschließend wurden die Platten erneut, wie oben, gewaschen und zum Nachweis gebundenen Mausantikörpers mit Peroxidase-konjugiertem Kaninchen-anti-Maus Immunglobulin (Dynatech, Plochingen) für 90 Minuten inkubiert (50 µl; Verdünnung 1:1000). Nach erneutem Waschen erfolgte die Quantifizierung der gebundenen Peroxidase mittels ABTS (Boehringer, Mannheim). Pro Mikrotiterplattennapf wurden 50 µl der Substratlösung (2mg/ml ABTS, gelöst in 0,1 M Citrat-, 0,2 M Natriumphosphatpuffer, pH 4,6; der Puffer enthielt 0,0025% H₂O₂) zugegeben. Die Auswertung erfolgte photometrisch. Mit diesem Test wurden 8 mehr oder minder stark reagierende, positive Klone unter etwa 600 getesteten Klonen identifiziert.

### Beispiel 3: Eigenschaften der erfindungsgemäßen Antikörper

### a) Feinspezifität im indirekten ELISA

Nach einer zweifachen Klonierung wurde der Antikörperhaltige Kulturüberstand gewonnen und die Spezifität von zweien der Antikörper näher überprüft. Die Überprüfung erfolgte ebenfalls im sogenannten indirekten ELISA. Es wurden verschiedene Antigene (C3; C3a-desArg; RSA; KLH; RSA-OP; KLH-OP), wie oben beschrieben, an PVC-ELISA-Platten adsorbiert. Alle weiteren Nachweisschritte erfolgten wie oben beschrieben. Die beiden monoklonalen Antikörper reagierten gleichartig. Es zeigte sich eine deutliche Reaktion der monoklonalen Antikörper gegen C3a-desArg sowie gegen die Octapeptid-tragenden Proteine (z.B. RSA-OP). Keine Reaktion ergab sich gegen natives C3, gegen nicht substituiertes KLH oder nicht substituiertes Rinderserumalbumin oder in Abwesenheit von Antigen.

### b) Reaktionsmuster im "Western Blot"

Als Bestätigungstest wurde eine "Western-blot-Analyse" durchgeführt. Die Auftrennung der Proteine erfolgte mittels SDS-Polyacrylamidgelelektrophorese (Gradientengel von 5 - 18 %). Die Proteine wurden elektrophoretisch auf Nitrozellulosepapier transferiert. Nach Absättigung der Filter mit 1%iger RSA-Lösung wurden einzelne Nitrozellulosestreifen mit verschiedenen Antikörpern (Kulturüberstände) inkubiert. Die weiteren Behandlungsschritte entsprechen denen eines indirekten ELISA und sind deshalb nicht gesondert aufgeführt. Als Substrat wurde Chlor-1-naphthol (Sigma, Taufkirchen) verwendet. Die beiden Antikörper reagierten nach Auftragen von gereinigtem C3a mit einem Protein eines Molekulargewichts von 9000 - 10000 D, nicht aber mit C3. Die Antikörper erfassen somit eine neoantigene Determinante von C3a bzw. C3a-desArg.

### c) Epitopanalyse:

Zur Identifizierung des von den beiden erfindungsgemäßen monoklonalen Antikörpern erkannten Epitopes wurde ein kompetitiver ELISA durchgeführt. Verschiedene monoklonale Antikörper mit Spezifität für das Komplementfragment C3a wurden mit einem überschuß an Octapeptid vorinkubiert und anschließend wie oben beschrieben im indirekten ELISA eingesetzt. Eine Vorinkubation des Antikörpers mit einem etwa 10fachen überschuß an freiem Octapeptid blockiert nur die beiden erfindungsgemäßen monoklonalen Antikörper, d.h. es erfolgt dann keine Reaktion mehr mit C3a und/oder C3a-desArg. Die Reaktivität anderer Antikörper gegen C3a bleibt dagegen unbeeinflußt durch freies Octapeptid. Dies bedeutet, daß das von den erfindungsgemäßen Antikörpern erkannte Epitop innerhalb der 8 C-terminalen Aminosäuren von C3a-desArg liegen muß.

### d) Nachweis von C3a-desArg

Die Eignung der Anti-Octapeptid-Antikörper für eine Quantifizierung von C3a bzw. C3a-desArg wurde in einem sogenannten indirekten Sandwich-ELISA-System nachgewiesen. Hier wird das Antigen nicht direkt an die ELISA-Platte adsorbiert, sondern über ein polyklonales Serum, welches gegen C3a bzw. C3a-desArg gerichtet ist, aus der Antigen-haltigen Probe herausgefischt. An eine konventionelle ELISA-Testplatte wurde dazu die IgG-Fraktion eines polyklonalen Antiserums gegen C3a bzw. C3a-desArg angelagert. Die Inkubation erfolgte für 1-2 Stunden. Nach dem Absättigen der Platte mit Rinderserumalbumin und Waschen, wie üblich, wurde die C3a- bzw. C3a-desArg-haltige Probe zugefügt und für 1-2 Stunden inkubiert. Gebundenes C3a bzw. C3a-desArg wurde analog zum indirekten ELISA nachgewiesen.

Nach dem letzten Waschen wurde die Reaktion nach Zugabe des Substrates photometrisch quantifiziert (siehe dazu auch Abb. 1). Es zeigte sich eine Reaktion bei C3a- bzw. C3a-desArg-haltigen Proben, aber fehlende Reaktionen bei C3-haltigen Proben. Zymosan-aktiviertes Serum, welches einen hohen C3a- bzw. C3a-desArg-Gehalt hat (im Radioimmunoassay nicht direkt meßbar, aber im erfindungsgemäß verwendeten ELISA in der Größenordnung von ca.4000 ng/ml), ergab bis zu einer Verdünnung von 1:4050 eine deutliche Reaktion. EDTA-Plasma mit einem endogen C3a- bzw. C3a-desArg-Gehalt von 100-200 ng/ml ergab dagegen bei einer 1:100 Verdünnung nur eine schwache Reaktion.

Mit Hilfe der erfindungsgemäßen monoklonalen Antikörper läßt sich somit C3a bzw. C3a-desArg auch in Anwesenheit großer Mengen an C3 ohne aufwendige Probenvorbehandlung nachweisen.

## Patentansprüche

1. Peptid aus dem Komplementfaktor C3a, **dadurch gekennzeichnet,** daß es die Aminosäuresequenz H₂N-Arg-Ala-Ser-His-Leu-Gly-Leu-Ala-COOH besitzt.

2. Peptid nach Anspruch 1, **dadurch gekennzeichnet,** daß es durch Proteolyse des gereinigten Komplementfaktors C3a oder C3a-des Arg hergestellt wird.

3. Peptid nach Anspruch 1, **dadurch gekennzeichnet,** daß es durch chemische Synthese hergestellt wird.

4. Peptid nach Anspruch 1, **dadurch gekennzeichnet,** daß es gentechnisch hergestellt wird.

5. Konjugat aus einem Trägermolekül und einem Peptid, **dadurch gekennzeichnet,** daß es ein Peptid nach mindestens einem der Ansprüche 1-4 enthält.

6. Konjugat nach Anspruch 5, **dadurch gekennzeichnet,** daß das Trägermolekül ein synthetisches oder natürliches Polymer ist.

7. Konjugat nach Anspruch 5, **dadurch gekennzeichnet,** daß das Trägermolekül ein Protein ist, bevorzugt ein Albumin oder Hämocyanin.

8. Verwendung des Peptids nach mindestens einem der Ansprüche 1-4 zum Herstellen von Antikörpern.

9. Verwendung des Konjugats nach mindestens einem der Ansprüche 5-7 zum Herstellen von Antikörpern.

10. Antikörper gegen ein Peptid nach mindestens einem der Ansprüche 1-4 und/oder gegen ein Konjugat nach mindestens einem der Ansprüche 5-7, **dadurch gekennezeichnet,** daß sie durch Immunisieren eines Tieres mit einem Peptid nach mindestens einem der Ansprüche 1 bis 4 und/oder mit einem Konjugat nach mindestens einem der Ansprüche 5-7 erhältlich sind.

11. Antikörper nach Anspruch 10, **dadurch gekennzeichnet,** daß sie spezifisch mit einem Peptid nach mindestens einem der Ansprüche 1 bis 4 und/oder mit einem Konjugat nach mindestens einem der Ansprüche 5-7 und/oder mit natürlichem Komplementfaktor C3a und/oder Teilen davon reagieren.

12. Antikörper nach Anspruch 10 und/oder 11, **dadurch gekennzeichnet,** daß sie polyklonal sind.

13. Antikörper nach Anspruch 10 und/oder 11, **dadurch gekennzeichnet,** daß sie monoklonal sind.

14. Zell-Linie, **dadurch gekennzeichnet,** daß sie Antikörper nach Anspruch 13 produziert.

15. Zell-Linie nach Anspruch 14, **dadurch gekennzeichnet,** daß sie eine Säugetier-Zellinie ist.

16. Verwendung von Antikörpern nach mindestens einem der Ansprüche 10 bis 13, **dadurch gekennzeichnet,** daß die Antikörper spezifisch an die Komplementfaktoren C3a und/oder C3a-desArg gebunden werden und der Komplementfaktor-Antikörper-Komplex durch einen zweiten Antikörper detektiert wird.

17. Verwendung von Antikörpern nach Anspruch 16, **dadurch gekennzeichnet,** daß die Menge des gebundenen zweiten Antikörpers meßbar ist.

18. Verwendung von Antikörpern nach Anspruch 17, **dadurch gekennzeichnet,** daß an den zweiten Antikörper ein Markerenzym, bevorzugt Peroxidase, gebunden ist.

19. Verwendung von Antikörpern nach mindestens einem der Ansprüche 10 bis 13, **dadurch gekennzeichnet,** daß sie für die direkte Bestimmung von Komplementfaktor C3a und/oder C3a-desArg im Plasma und/oder Serum eingesetzt werden.

20. Verwendung von Antikörpern nach mindestens einem der Ansprüche 10 bis 13 zur Herstellung eines Medikaments zur Behandlung von Krankheiten, die Folge einer Überaktivierung des Komplementsystems sind.

21. Antikörper nach mindestens einem der Ansprüche 10 bis 13 zur Verwendung als Arzneimittel.

## Claims

1. Peptide from the complement factor C3a, characterised in that it possesses the amino acid sequence H₂N-Arg-Ala-Ser-His-Leu-Gly-Leu-Ala-COOH.

2. Peptide according to Claim 1, characterised in that it is prepared by proteolysis of the purified complement factor C3a or of des-Arg C3a.

3. Peptide according to Claim 1, characterised in that it is prepared by chemical synthesis.

4. Peptide according to Claim 1, characterised in that it is prepared by gene technology.

5. Conjugate of a carrier molecule and a peptide, characterised in that it contains a peptide according to at least one of Claims 1-4.

6. Conjugate according to Claim 5, characterised in that the carrier molecule is a synthetic or natural polymer.

7. Conjugate according to Claim 5, characterised in that the carrier molecule is a protein, preferably an albumin or haemocyanin.

8. Use of the peptide according to at least one of Claims 1-4 for preparing antibodies.

9. Use of the conjugate according to at least one of Claims 5-7 for preparing antibodies.

10. Antibodies against a peptide according to at least one of Claims 1-4 and/or against a conjugate according to at least one of Claims 5-7, characterised in that they can be obtained by immunising an animal with a peptide according to at least one of Claims 1 to 4 and/or with a conjugate according to at least one of Claims 5-7.

11. Antibodies according to Claim 10, characterised in that they react specifically with a peptide according to at least one of Claims 1 to 4 and/or with a conjugate according to at least one of Claims 5-7 and/or with natural complement factor C3a and/or parts thereof.

12. Antibodies according to Claim 10 and/or 11, characterised in that they are polyclonal.

13. Antibodies according to Claim 10 and/or 11, characterised in that they are monoclonal.

14. Cell line, characterised in that it produces antibodies according to Claim 13.

15. Cell line according to Claim 14, characterised in that it is a mammalian cell line.

16. Use of antibodies according to at least one of Claims 10 to 13, characterised in that the antibodies are bound specifically to the complement factors C3a and/or des-Arg C3a and the complement factor-antibody complex is detected by a second antibody.

17. Use of antibodies according to Claim 16, characterised in that the quantity of the bound second antibody is measurable.

18. Use of antibodies according to Claim 17, characterised in that a marker enzyme, preferably peroxidase, is bound to the second antibody.

19. Use of antibodies according to at least one of Claims 10 to 13, characterised in that they are employed for the direct determination of complement factor C3a and/or des-Arg C3a in plasma and/or serum.

20. Use of antibodies according to at least one of Claims 10 to 13 for preparing a medicament for the treatment of diseases which are a consequence of a hyperactivation of the complement system.

21. Antibodies according to at least one of Claims 10 to 13 for use as a medicament.

## Revendications

1. Peptide provenant du facteur du complément C3a, caractérisé en ce qu'il possède la séquence d'aminoacides H₂N-Arg-Ala-Ser-His-Leu-Gly-Leu-Ala-COOH.

2. Peptide selon la revendication 1, caractérisé en ce qu'il est obtenu par protéolyse du facteur du complément C3a ou C3a-desArg purifié.

3. Peptide selon la revendication 1, caractérisé en ce qu'il est préparé par synthèse chimique.

4. Peptide selon la revendication 1, caractérisé en ce qu'il est produit par génie génétique.

5. Conjugué d'une molécule porteuse et d'un peptide, caractérisé en ce qu'il contient un peptide selon au moins l'une des revendications 1 à 4.

6. Conjugué selon la revendication 5, caractérisé en ce que la molécule porteuse est un polymère naturel ou synthétique.

7. Conjugué selon la revendication 5, caractérisé en ce que la molécule porteuse est une protéine, de préférence une albumine ou une hémocyanine.

8. Utilisation du peptide selon au moins l'une des revendications 1 à 4, pour la production d'anticorps.

9. Utilisation du conjugué selon au moins l'une des revendications 5 à 7, pour la production d'anticorps.

10. Anticorps dirigés contre un peptide selon au moins l'une des revendications 1 à 4 et/ou contre un conjugué selon au moins l'une des revendications 5 à 7, caractérisés en ce qu'ils peuvent être obtenus par immunisation d'un animal avec un peptide selon au moins l'une des revendications 1 à 4 et/ou avec un conjugué selon au moins l'une des revendications 5 à 7.

11. Anticorps selon la revendication 10, caractérisés en ce qu'ils réagissent spécifiquement avec un peptide selon au moins l'une des revendications 1 à 4 et/ou avec un conjugué selon au moins l'une des revendications 5 à 7 et/ou avec le facteur du complément C3a naturel et/ou des parties de celui-ci.

12. Anticorps selon la revendication 10 et/ou 11, caractérisés en ce qu'ils sont polyclonaux.

13. Anticorps selon la revendication 10 et/ou 11, caractérisés en ce qu'ils sont monoclonaux.

14. Lignée cellulaire, caractérisée en ce qu'elle produit des anticorps selon la revendication 13.

15. Lignée cellulaire selon la revendication 14, caractérisée en ce qu'elle est une lignée cellulaire de mammifère.

16. Utilisation d'anticorps selon au moins l'une des revendications 10 à 13, caractérisée en ce que les anticorps sont liés spécifiquement aux facteurs du complément C3a et/ou C3a-desArg, et le complexe anticorps-facteur du complément est détecté au moyen d'un second anticorps.

17. Utilisation d'anticorps selon la revendication 16, caractérisé en ce que la quantité du second anticorps lié est mesurable.

18. Utilisation d'anticorps selon la revendication 17, caractérisée en ce qu'une enzyme de marquage, de préférence la peroxydase, est liée au second anticorps.

19. Utilisation d'anticorps selon au moins l'une des revendications 10 à 13, caractérisée en ce qu'ils sont utilisés pour la détermination directe du facteur du complément C3a et/ou C3a-desArg dans le plasma et/ou le sérum.

20. Utilisation d'anticorps selon au moins l'une des revendications 10 à 13, pour la fabrication d'un médicament destiné au traitement de maladies qui sont la conséquence d'une suractivation du système du complément.

21. Anticorps selon au moins l'une des revendications 10 à 13, pour utilisation en tant que médicament.
